**Europäisches Patentamt**

(19) **European Patent Office**

**Office européen des brevets**

(11) Veröffentlichungsnummer: **0 040 715**
**B1**

(12) ## EUROPÄISCHE PATENTSCHRIFT

(45) Veröffentlichungstag der Patentschrift:
**21.09.83**

(51) Int. Cl.³: **C 07 C 47/09**, C 07 C 27/32,
C 07 C 31/04, C 07 C 69/14

(21) Anmeldenummer: **81103347.1**

(22) Anmeldetag: **04.05.81**

(54) **Verfahren zur Gewinnung von Acetaldehyd und Methanol aus Reaktionsgemischen der Methanol-Homologisierung.**

(30) Priorität: **23.05.80 DE 3019765**

(43) Veröffentlichungstag der Anmeldung:
**02.12.81 Patentblatt 81/48**

(45) Bekanntmachung des Hinweises auf die Patenterteilung:
**21.09.83 Patentblatt 83/38**

(84) Benannte Vertragsstaaten:
**BE DE FR GB IT NL**

(56) Entgegenhaltungen:
**DE-B-1 046 603**
**DE-B-1 418 280**
**GB-A-738 654**
**US-A-4 201 868**

(73) Patentinhaber: **BASF Aktiengesellschaft,
Carl-Bosch-Strasse 38, D-6700 Ludwigshafen (DE)**

(72) Erfinder: **Hochstein, Waldhelm, Dr., Am Wurmberg 4,
D-6713 Freinsheim (DE)**
Erfinder: **Kaibel, Gerd, Robert-Bosch-Strasse 4,
D-6840 Lampertheim (DE)**
Erfinder: **Müller, Franz-Josef, Dr., Müller-Thurgau-Weg 1,
D-6706 Wachenheim (DE)**
Erfinder: **Hartmann, Horst, Lindenstrasse 45,
D-6737 Boehl-Iggelheim (DE)**

Verfahren zur Gewinnung von Acetaldehyd und Methanol aus Reaktionsgemischen der
Methanol-Homologisierung

Die vorliegende Erfindung betrifft ein Verfahren zur Gewinnung von Acetaldehyd und Methanol aus Reaktionsgemischen, die bei der Homologisierung von Methanol anfallen.

Unter Homologisierung versteht man die Umsetzung von Alkoholen mit Kohlenmonoxid und Wasserstoff bei erhöhter Temperatur und erhöhtem Druck in Gegenwart von Metallcarbonylkomplexen, beispielsweise von Cobaltcarbonylkomplexen. Nähere Angaben zu dieser Reaktion sind der Monographie von Falbe, «Chemierohstoffe aus Kohle», Georg-Thieme-Verlag, 1977, Seite 329 ff zu entnehmen.

Im Falle der Homologisierung von Methanol erhält man Gemische zahlreicher Stoffe, deren Anteile von den Reaktionsbedingungen abhängig sind. Die wichtigsten der in grösseren Mengen anfallenden Komponenten dieser Gemische sind hierbei der Ausgangsstoff Methanol und die Homologisierungsprodukte Ethanol, Acetaldehyd, Acetalaldehyddimethylacetal, Essigsäure, Methylacetat und Wasser. Arbeitet man auf relativ geringe Umsätze hin, so wird die Ethanolbildung weitgehend unterdrückt und man erhält Gemische die im wesentlichen aus folgenden Stoffen bestehen:

30–75 Gew.-% Methanol
2–10 Gew.-% Acetaldehyd
8-30 Gew.-% Acetaldehyddimethylacetal
5–10 Gew.-% Methylacetat und
10–20 Gew.-% Wasser.

Derartige, an Acetaldehyd reichen Gemische sind in aller Regel zu bevorzugen, da der Acetaldehyd vielseitiger verwendbar ist als das bei hohen Umsätzen hauptsächlich erhältliche Ethanol. Allerdings bereitet die destillative Aufbereitung solcher Gemische erhebliche Schwierigkeiten, weil das Acetataldehyddimethylacetal zur Gewinnung des Acetataldehyds gespalten werden muss und weil sowohl Actataldehyddimethylacetal als auch Methylacetat mit Methanol Azeotrope bilden, so dass das Methanol deshalb nicht ohne weiteres zurückgewonnen werden kann.

Aus der DE-B 1 046 603 ist es bekannt, Ethylacetat aus seinen Gemischen mit Wasser, Acetaldehyd, Acetaldehyddiethylacetal und Essigsäure destillativ unter Acetalspaltung zu gewinnen, wobei der Acetaldehyd zusammen geringeren Anteilen der übrigen Komponenten als Kopfprodukt abgezogen wird, während der Rest des Gemisches im Sumpf anfällt und einer weiteren Fraktionierung mit dem Ziel der Gewinnung reinen Ethylacetats bedarf. Für die Lösung des Problems, die besonders schwierig zu zerlegenden Reaktionsgemische aus der Methanol-Homologisierung in ihre Bestandteile zu trennen, bietet die DE-A 1 046 603 jedoch keinen Anhaltspunkt.

Aus der DE-B 1 418 280 ist es weiterhin bekannt, Gemische aus überwiegenden Mengen Methylacetat, untergeordneten Mengen Methanol und Wasser sowie sehr geringen Mengen Acetaldehyd zur Reduzierung des Methanol- und Wasseranteils zunächst einer Extraktivdestillation mit Wasser zu unterwerfen, wonach man von dem als Kopfprodukt verbleibenden Gemisch in einer zweiten Kolonne geringe Mengen von Acetaldehyd und Methylacetat abdestilliert. Die Abtrennung des Acetaldehyds gelingt auf diese Weise jedoch nur zu etwa 27%, genügt aber der Zielsetzung dieses Verfahrens, nämlich eine Methylacetatqualität zu erhalten, welche die störungsfreie Hydrolyse zu Methanol und Essigsäure gestattet. Darüber, wie man die Ausgangsgemische in ihre reinen Komponenten zerlegen könnte, gibt die DE-A 1 418 280 indes keinen Aufschluss.

Schliesslich ist es auch der GB-A 738 654 bekannt, Alkohole und Aldehyde, wie sie bei der Oxoreaktion entstehen, also $C_n$-Alkohole und $C_n$-Aldehyde, wobei n mindestens 3 ist, durch Extraktivdestillation mittels saurer wässriger Lösung bestimmter wasserlöslicher aromatischer Verbindungen wie Na-Xylolsulfonat voneinander zu trennen. Auch dieser Vorveröffentlichung ist kein Hindernis darauf zu entnehmen, wie das Destillationsproblem gemäss der vorliegenden Erfindung zu bewältigen wäre.

Der Erfindung lag daher die Aufgabe zugrunde, den bei der Homologisierung von Methanol in freier oder in Form des Dimethylacetals gebundener Form anfallenden Acetataldehyd auf möglichst wirtschaftliche und technisch einfache Weise destillativ von den Homologisierungsgemischen abzutrennen und gleichzeitig das Methanol zurückzugewinnen.

Es wurde nun gefunden, dass man Acetaldehyd und Methanol aus Reaktionsgemischen, die bei der Homologisierung von Methanol anfallen und die neben Acetaldehyd im wesentlichen Acetaldehyddimethylacetal, Methanol, Methylacetat und Wasser enthalten, auf wirtschaftliche und technisch einfache Weise destillativ gewinnen kann, wenn man

a) das Reaktionsgemisch bei einer Temperatur in den mittleren Teil einer Kolonne I gibt, bei welcher das Methylacetat oder sein azeotropes Gemisch mit Methanol, nicht aber das Methanol gänzlich verdampft,

$b_1$) Im Gegenstrom zu den aufsteigenden Dämpfen flüssige wässrige Säure leitet, die einem oberen Viertel der Kolonne I zugeführt wird und welche eine Spaltung des Acetaldehyddimethylacetals in Acetaldehyd und Methanol bewirkt, oder

$b_2$) anstelle der wässrigen Säure lediglich Wasser verwendet und die Acetalspaltung mittels eines festen sauren Ionenaustauschers bewirkt,

c) vom Kopf der Kolonne I ein dampfförmiges Gemisch aus Methylacetat und Acetaldehyd abzieht, welches in einer Kolonne II in seine Komponenten zerlegt wird,

d) in einem unteren Viertel der Kolonne I ein flüssiges Gemisch aus Methanol und Wasser abzieht, welches in einer Kolonne III in seine Komponenten zerlegt wird, und wenn man

e) einen Teil der im Sumpf der Kolonne I anfallenden wässrigen Säure (Variante b$_1$) bzw. des Wassers (Variante b$_2$), zusammen mit untergeordneten Mengen sonstiger Produkte aus dem System entfernt und den restlichen Teil dieser wässrigen Säure bzw. des Wassers mit dem im Sumpf der Kolonne III anfallenden Wasser vereinigt und die vereinigte wässrige Lösung nach Zugabe einer Säure wieder in das obere Viertel der Kolonne I zurückführt.

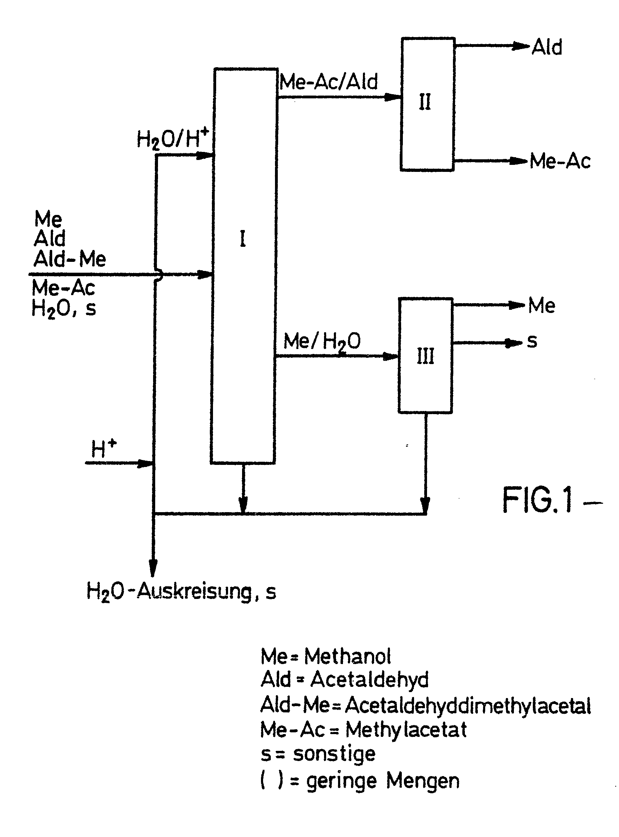

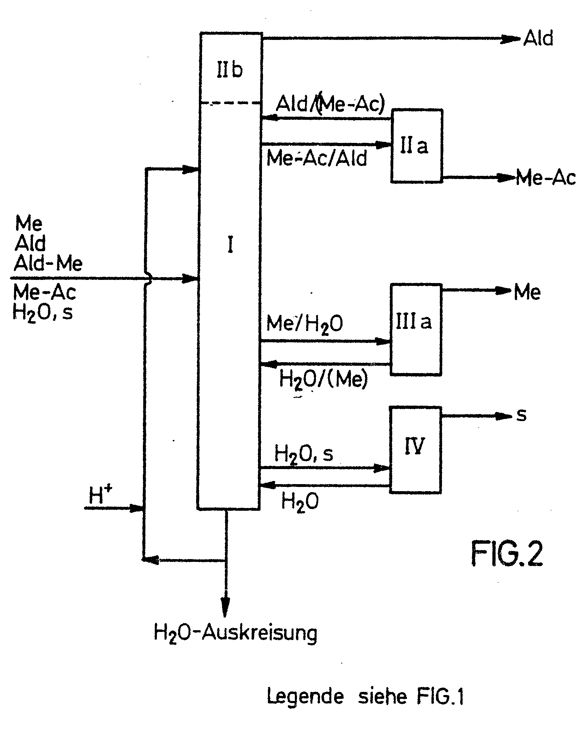

Das erfindungsgemässe Verfahren unter Anwendung der Variante (b$_1$) wird durch Figur 1 veranschaulicht, die aufgrund der vorstehenden Verfahrensdefinition aus sich selbst heraus verständlich ist und deshalb keiner weiteren Erläuterung bedarf.

Figur 2 zeigt verschiedene Ausgestaltungen dieser Verfahrensweise, die darin bestehen, dass man, verglichen mit der Grundanordnung gemäss Figur 1

- die Kolonne II zu einer reinen Abtriebskolonne IIa verkürzt und dafür die Kolonne I um den Teil IIb nach oben verlängert und dadurch die Acetaldehyd-Methylacetatfraktionierung in die Hautkolonne I verlegt, und/oder

- die Kolonne III zu einer reinen Verstärkungskolonne IIIa verkürzt und dadurch die Wasser-Methanol-Fraktionierung ebenfalls in die Hauptkolonne I verlagert, und/oder dass man

- die sonstigen Verfahrensprodukte wie Ethanol, C$_3$- und C$_4$-Alkohole und -Aldehyde durch Destillation in einer Kolonne IV vom Wasser abtrennt.

Führt man das erfindungsgemässe Verfahren bei Normaldruck aus, so herrscht, entsprechend den Siedepunkten der beteiligten Stoffe und Azeotrope im Sumpf der Kolonne I eine Temperatur von 100–120°C, in der Mitte eine Temperatur von 60–75°C und am Kopf eine Temperatur von 21–35°C. Die Temperaturprofile der übrigen Kolonne II und III bzw. IIa, IIIa und IV ergeben sich hieraus entsprechend.

Da der Acetaldehyd jedoch bei 21°C siedet und deshalb nicht mit normalem Kühlwasser kondensiert werden kann, wird das Arbeiten unter einem Druck von 2–4 bar bevorzugt, unter welchem der Acetaldehyd bei 40–65°C siedet und deshalb mit normalem Kühlwasser kondensiert werden kann.

Die Kolonne I umfasst zweckmässigerweise 25–60, die Kolonne II 5–15, die Kolonne III 10–30 und die Kolonne IV 10–30 theoretische Böden. Macht man von den Varianten bezüglich der Methylacetat- und Methanol-Gewinnung gemäss Figur 2 Gebrauch, so verwendet man Kolonne IIa bzw. IIIa mit 5–10 bzw. 8–30 theoretischen Böden, und die Zahl der theoretischen Böden der Kolonne I erhöht sich entsprechend um 3–10 Böden nach oben (IIb).

Auf die Bauart der Kolonne kommt es nicht an, so dass man vorzugsweise die billigen Füllkörperkolonne verwendet.

Das erfindungsgemässe Verfahren ist prinzipiell von der Homologisierungsreaktion unabhängig, d.h. es würde auch mit hohen Ethanol-Anteilen gelingen. In diesem Fall wäre dann lediglich die Destillation in der Kolonne IV unerlässlich. Da es aber entsprechend der Zielsetzung auf die wirtschaftliche Gewinnung hauptsächlich des Acetaldehyds ankommt, wird man die Homologisierung so vornehmen, dass sich nur wenig Ethanol bildet. Dies kann man erreichen, wenn das Synthesegas nicht mehr als 55 Vol.% Wasserstoff enthält und wenn man den Methanolumsatz auf etwa 40% begrenzt. Im übrigen kann man die Homologisierung wie üblich vornehmen, d.h. unter einem Druck von 100 bis 700 bar, bei 150–210°C und in Gegenwart von 0,1 bis 2,0 Gew.-%, bezogen auf die Gesamtmenge des Reaktionsgemisches im Reaktor, eines Homologisierungskatalysators.

Solche Katalysatoren sind Carbonylkomplexe von Metallen der VIII. Gruppe des Periodensystems sowie auch von Rhenium. Allgemein eignen sich hier vor allem diejenigen Verbindungen, die man auch für die Hydroformylierung verwendet, also vor allem Rhodium- und Cobaltkomplexe. Man kann die Carbonylkomplexe wie CO$_2$(CO)$_8$ einsetzen oder vorzugsweise Verbindungen der betreffenden Metalle wie Rhodiumoxid, Rhodiumacetat oder Cobaltacetat, da sich die Komplexe unter den Reaktionsbedingungen in situ bilden. Die Stabilität der Komplexe kann durch weitere Liganden wie Acetylaceton, tertiäre Phosphine oder Phosphite erhöht werden. Sind die Metallkomplexe schwer flüchtig, so verbleiben sie im Reaktor, wenn man das Reaktionsgemisch gasförmig entnimmt. Sind sie dagegen flüchtig, so muss der Katalysator wie üblich in einem gesonderten Verfahrensschritt, beispielsweise durch Behandlung mit einer wässrigen Säure zerstört werden, wonach man das Reaktionsgemisch von den anorganischen Bestandteilen abdestilliert. Eine andere Möglichkeit besteht in der oxidativen Zerstörung der Komplexe durch Behandlung mit Luft.

Wesentlich für das erfindungsgemässe Verfahren sind die Verfahrensschritte (b$_1$) bzw. (b$_2$), also die Spaltung des Acetaldehyddimethylacetals mittels einer Säure in der oberen Hälfte der Kolonne I. Da hier nur wenig überschüssiges Methanol vorhanden ist und der leichtflüchtige Acetaldehyd sofort nach oben entweicht, kann sich das Acetal nicht wieder zurückbilden, wie es der Fall wäre, wenn man die Acetalspaltung etwa im Sumpf der Kolonne vornehmen würde.

Als Säuren eignen sich beliebige starke Säuren, beispielsweise Schwefelsäure, Salzsäure, Salpetersäure, Ameisensäure und Sulfonsäuren wie p-Toluolsulfonsäure in Konzentrationen von etwa 0,01–10 Gew.-%.

Da bei der Synthese laufend Wasser gebildet wird, muss stets ein Teil des Wassers aus dem Wasserkreislauf der Kolonne I entfernt («ausgekreist») werden, wobei auch der entsprechende Teil der Säure und gegebenenfalls auch die sonstigen Produkte, sofern die Destillation IV nicht vorgesehen ist, verloren geht. Die Säure muss daher laufend um den mit dem Auskreisungswasser verlorenen Anteil ergänzt werden, wie aus den Figuren ersichtlich ist.

Ein Verbrauch von Säure findet gemäss Variante (b₂) Ausführungsform nicht statt, wenn man als Säure einen Ionenaustauscher verwendet, den man im Mittelteil der Kolonne I anordnen kann. Verfahrenstechnisch vorteilhafter ist es jedoch, das in der Kolonne I befindliche Gemisch ausserhalb der Kolonne mit dem Ionenaustauscher in Berührung zu bringen, indem man an einer oder mehreren Stellen einen Teil des Gemisches abzieht, ausserhalb der Kolonne über den Ionenaustauscher leitet und jeweils auf Höhe der Entnahmestelle wieder in die Kolonne zurückführt. Der Wasserkreislauf wird von der Verwendung der Ionenaustauscher nicht berührt, weil das Wasser für die Acetalspaltung benötigt wird.

Besonders bemerkenswert ist der Verfahrensschritt (c). Es war nämlich zu erwarten, dass am Kopf der Kolonne I das Azeotrop aus Methylacetat und Methanol (Sdp. 53°C, Normaldruck) auftritt und dass diese beiden Stoffe ohne erheblichen Aufwand nicht mehr voneinander getrennt werden könnten. Durch den Acetaldehyd wird dieses Azeotrop jedoch gebrochen, so dass man als Kopfprodukt ein Gemisch aus Acetaldehyd und Methylacetat erhält, dessen Auftrennung in der Kolonne II unproblematisch ist. Geringe Mengen Methanol, die u.U. ebenfalls am Kopf der Kolonne noch anfallen können, können mittels der Verfahrensvariante gemäss Figur 2 entfernt werden, da das Kopfprodukt der Kolonne IIa, das aus Acetaldehyd, aus etwas Methylacetat und in diesem Fall aus etwas Methanol besteht, in die Kolonne I zurückgegeben wird.

Da mittels des erfindungsgemässen Verfahrens das Methanol/Methylacetatazeotrop gebrochen wird, bereitet die Rückgewinnung des Methanols aus seinem Gemisch mit Wasser keine Schwierigkeiten mehr. Deshalb erübrigen sich weitere Ausführungen zum Verfahrensschritt (d), welcher die Spaltung des Methanol/Wasser-Gemisches betrifft. Das gleiche gilt für den Verfahrensschritt (e) sowie die Verfahrensvariante, gemäss der die sonstigen Produkte, will man sie nicht mit dem Auskreisungswasser verwerfen, als Destillat der zusätzlichen Kolonne IV gewonnen werden.

Beispiel

Dieses Beispiel wurde in einer Versuchsapparatur gemäss Figur 1 unter Normaldruck ausgeführt. Sämtliche Kolonnen waren als Füllkörperkolonnen ausgebildet. Die Kolonne I war 2 m hoch, hatte einen inneren Durchmesser von 5 cm und 40 theoretische Böden. Die Kolonne II war 1 m hoch, hatte einen Innendurchmesser von 5 cm und 20 theoretische Böden und die Kolonne III war 0,8 m hoch, hatte einen Innendurchmesser von 5 cm und 12 theoretische Böden. Auf Höhe des 30. theoretischen Bodens (von unten gezählt), bei welchem die Temperatur 61°C betrug, wurden der Kolonne I stündlich 300 g eines Reaktionsgemisches zugeführt, welches der Homologisierung von Methanol entstammte.

Die Homologisierung wurde bei 300 bar und 125°C in Gegenwart von 0,3 Gew.-% Cobalt als Cobaltacetat, bezogen auf die Menge des Reaktorinhalts, mit einem Gemisch aus 50 Vol.% Wasserstoff und 50 Vol.% Kohlenmonoxid vorgenommen, wobei ein Methanolumsatz von etwa 26% eingehalten wurde. Das erhaltene Reaktionsgemisch wurde nach Entspannung mit Luft begast, wobei der Cobaltkomplex zerstört wurde. Anschliessend wurde das Gemisch von dem entstandenen Cobaltsalz abdestilliert. Dieses Gemisch hatte folgende Zusammensetzung:

54 Gew.-% (162 g) Methanol
7 Gew.-% (21 g) Acetaldehyd
9 Gew.-% (27 g) Acetaldehyddimethylacetal
7 Gew.-% (21 g) Methylacetat
15 Gew.-% (45 g) Wasser
6 Gew.-% (18 g) Äthanol
2 Gew.-% (6 g) sonstige Produkte.

Auf der Höhe des 35. Bodens (48°C) wurden stündlich 100 g 1 gew.-%ige wässrige Schwefelsäure, die noch etwas Ethanol und sonstige Produkte enthielt, in die Kolonne I eingeführt.

Bei einem Rücklaufverhältnis von 5 wurden stündlich 34 g Acetaldehyd, 21 g Methylacetat, 0,5 g Acetaldehyddimethylacetal und noch 0,1 g Methanol am Kolonnenkopf (29°C) abgezogen. Dieses Gemisch wurde in der Kolonne II in Acetaldehyd (Ausbeute praktisch 100%) und das nur geringfügig verunreinigte Methylacetat getrennt. Auf Höhe des 15. Bodens (75°C) wurde der Kolonne I ein dampfförmiger Seitenabzug entnommen, der in der Kolonne III in 181 g Methanol als Kopfprodukt und 64 g Sumpfprodukt, das aus 40 g Wasser, 18 g Ethanol und 6 g sonstiger Produkte bestand, zerlegt wurde.

Dieses Sumpfprodukt wurde mit dem Sumpfprodukt der Kolonne I (110°C), welches aus 100 g der wässrigen Schwefelsäure bestand, vereinigt, wonach 64 g der vereinigten Sumpfprodukte aus dem Kreislauf entfernt wurden. Anschliessend wurde zur Konstanthaltung der Säurekonzentration wieder etwas Schwefelsäure in den Kreislauf gegeben.

Patentansprüche

1. Verfahren zur Gewinnung von Acetaldehyd und Methanol aus Reaktionsgemischen, die bei der Homologisierung von Methanol anfallen und die neben Acetaldehyd im wesentlichen Acetaldehyddimethylacetal, Methanol, Methylacetat und Wasser enthalten, dadurch gekennzeichnet, dass man

a) Das Reaktionsgemisch bei einer Temperatur in den mittleren Teil einer Kolonne I gibt, bei welcher das Methylacetat oder sein azeotropes Gemisch mit Methanol, nicht aber das Methanol gänzlich verdampft,

b₁) im Gegenstrom zu den aufsteigenden Dämpfen eine flüssige wässrige Säure leitet, die einem oberen Viertel der Kolonne I zugeführt wird und welche eine Spaltung des Acetaldehyddimethylacetals in Acetaldehyd und Methanol bewirkt, oder

b₂) anstelle der wässrigen Säure lediglich Wasser verwendet und die Acetalspaltung mittels eines festen sauren Ionenaustauschers bewirkt,

c) vom Kopf der Kolonne I ein dampfförmiges Gemisch aus Methylacetat und Acetaldehyd abzieht, welches in einer Kolonne II in seine Komponenten zerlegt wird,

d) in einem unteren Viertel der Kolonne I ein flüssiges Gemisch aus Methanol und Wasser abzieht, welches in einer Kolonne III in seine Komponenten zerlegt wird und dass man

e) einen Teil der im Sumpf der Kolonne I anfallenden wässrigen Säure (Variante b₁) bzw. des Wassers (Variante b₂), zusammen mit untergeordneten Mengen sonstiger Produkte aus dem System entfernt und den restlichen Teil dieser wässrigen Säure bzw. des Wassers mit dem im Sumpf der Kolonne III anfallenden Wasser vereinigt und die vereinigte wässrige Lösung nach Zugabe einer Säure wieder in das obere Viertel der Kolonne I zurückführt.

2. Verfahren nach Anspruch 1, dadurch gekennzeichnet, dass man anstelle der Kolonne II eine Abtriebskolonne IIa verwendet, in welche man das Acetaldehyd/-Methylacetat-Gemisch in den oberen Teil eingibt, das an Methylacetat abgereicherte, hauptsächlich Acetaldehyd enthaltende Gemisch vom Kopf der Kolonne IIa wieder in die Kolonne I zurückführt, den Grossteil des Methylacetates aus dem Kolonnensumpf von IIa entnimmt und die Trennung des in die Kolonne I zurückgeführten Methylacetat/Acetaldehyd-Gemisches in einem zusätzlich auf die Kolonne I aufgesetzten Kolonnenteil IIb vornimmt.

3. Verfahren nach den Ansprüchen 1 und 2, dadurch gekennzeichnet, dass man die Kolonne III durch eine Verstärkungskolonne IIIa ersetzt, in welche man das Methanol/Wasser-Gemisch in den unteren Teil eingibt, das an Methanol abgereicherte, hauptsächlich Wasser enthaltende Gemisch vom Sumpf der Kolonne IIIa wieder in die Kolonne I zurückführt und den Grossteil des Methanols dem Kolonnenkopf von IIIa entnimmt.

4. Verfahren nach den Ansprüchen 1 bis 3, dadurch gekennzeichnet, dass man die sonstigen Produkte, die sich im Gemisch mit der wässrigen Säure bzw. mit dem Wasser im unteren Teil der Kolonne I anreichern, in einer Seitenstromdestillation in einer Kolonne IV vom Wasser abtrennt.

## Claims

1. A process for isolating acetaldehyde and methanol from a reaction mixture which results from the homogenization of methanol and which in addition to acetaldehyde essentially contains acetaldehyde dimethylacetal, methanol, methyl acetate and water, wherein

a) the reaction mixture is introduced into the middle zone of a column I, at a temperature at which the methyl acetate and its azeotropic mixture with methanol vaporize completely, but the methanol does not,

b₁) a liquid aqueous acid, fed into the upper quarter of the column I, is led in counter-current to the rising vapors and causes cleavage of the acetaldehyde-dimethylacetal into acetaldehyde and methanol, or

b₂) instead of the aqueous acid, water alone is used, and the acetal cleavage is effected by means of a solid acidic ion exchanger,

c) a vapor mixture of methyl acetate and acetaldehyde is taken off at the top of column I and separated into its components in a column II,

d) in the lower quarter of column I, a liquid mixture of methanol and water is taken off and is separated into its components in a column III, and

e) a part of the aqueous acid (embodiment b₁) or of the water (embodiment b₂) arising in the bottom of column I is removed, together with minor amounts of other products, from the system, and the remainder of this aqueous acid or of this water is combined with the water forming in the bottom of column III and the combined aqueous solution, after mixing with an acid, is recycled into the upper quarter of column 1.

2. A process as claimed in claim 1, wherein, in place of column II, a stripping column IIa is used, in which the acetaldehyde/methyl acetete mixture is fed into the upper zone, the mixture depleted in methyl acetate and containing principally acetaldehyde is taken from the top of column IIa and returned to column I, the greater part of the methyl acetate is taken from the bottom of column IIa and the separation of the methyl acetate/acetaldehyde mixture returned to column I is effected in an additional column section IIb attached to the top of column I.

3. A process as claimed in claims 1 and 2, wherein column III is replaced by a rectifying column IIIa, in which the methanol/water mixture is introduced into the lower zone, the mixture depleted in methanol and principally containing water is returned from the bottom of column IIIa to column I, and the greater part of the methanol is taken off the top of column IIIa.

4. A process as claimed in claims 1 to 3, wherein the other products, which accumulate, as a mixture with the aqueous acid or with water, in the lower zone of column I, are separated from water by distilling a side stream in a column IV.

## Revendications

1. Procédé pour la récupération d'acétaldéhyde et de méthanol à partir de mélanges réactionnels qui résultent de l'homologuisation de méthanol et qui contiennent essentiellement, outre l'acétaldéhyde, du diméthylacétal, du méthanol, de l'acétate de méthyle et de l'eau, caractérisé en ce que

a) on introduit le mélange réactionnel dans la partie moyenne d'une colonne I à une température à laquelle l'acétate de méthyle ou son mélange azéotropique avec le méthanol, mais non le méthanol, s'évapore complètement,

b₁) on fait passer, à contre-courant par rapport aux vapeurs qui montent, un acide aqueux liquide qui est introduit dans le quart supérieur de la colonne I et qui provoque une dissociation du diméthylacétal d'acétaldéhyde en acétaldéhyde et en méthanol, ou

b₂) on utilise, à la place de l'acide aqueux, simplement de l'eau et on effectue la dissociation de l'acétal au moyen d'un échangeur d'ions acide solide,

c) on extrait, en tête de la colonne I, un mélange à l'état de vapeur d'acétate de méthyle et d'acétaldéhyde, mélange qui est décomposé en ses constituants dans une colonne II,

d) on extrait, dans le quart inférieur de la colonne I, un mélange liquide de méthanol et d'eau qui est décomposé en ses constituants dans une colonne III, et

e) on élimine du système une partie de l'acide aqueux (variante b₁) ou de l'eau (variante b₂) présent au bas de la colonne I, avec des quantités mineures d'autres produits, on réunit la partie restante de cet acide aqueux ou de l'eau à l'eau obtenue au bas de la colonne III et, après addition d'un acide, on renvoie la solution aqueuse ainsi réunie dans le quart supérieur de la colonne I.

2. Procédé selon la revendication 1, caractérisé en ce qu'on utilise, à la place de la colonne II, une colonne de rectification IIa, dans la partie supérieure de laquelle on introduit le mélange d'acétaldéhyde/acétate de méthyle, en ce que le mélange appauvri en acétate de méthyle et contenant principalement l'acétaldéhyde, obtenu en tête de la colonne IIa, est renvoyé dans la colonne I, en ce que la majeure partie de l'acétate de méthyle est extrait du bas de la colonne IIa et en ce que la séparation du mélange, d'acétate de méthyle/acétaldéhyde, renvoyé dans la colonne I, est effectuée dans une partie de colonne supplémentaire IIb, placée sur la colonne I.

3. Procédé selon la revendication 1 et 2, caractérisé en ce qu'on remplace la colonne III par une colonne de concentration IIIa, dans la partie inférieure de laquelle on introduit le mélange de méthanol/eau, en ce que le mélange appauvri en méthanol et contenant principalement de l'eau, obtenu au bas de la colonne IIIa, est renvoyé dans la colonne I et en ce que la majeure partie du méthanol est prélevée en tête de la colonne IIIa.

4. Procédé selon l'une quelconque des revendications 1 à 3, caractérisé en ce qu'on sépare de l'eau, par une distillation de produit de soutirage latéral dans une colonne IV, les autres produits qui s'enrichissent dans le mélange avec l'acide aqueux ou avec l'eau dans la partie inférieure de la colonne I.

FIG.1 –

H₂O-Auskreisung, s

Me = Methanol
Ald = Acetaldehyd
Ald-Me = Acetaldehyddimethylacetal
Me-Ac = Methylacetat
s = sonstige
( ) = geringe Mengen

FIG.2

Legende siehe FIG.1